# EUROPEAN PATENT APPLICATION

(11) **EP 1 275 656 A1**
(43) Date of publication of application: **15.01.2003**
(21) Application number: 01116917.4
(22) Date of filing: 11.07.2001
(51) Int. Cl.: C07J 71/00

(54) **Preparation of 9alpha-fluoro-11beta-hydroxypregnane 16,17-acetal derivatives**

(71) Applicant: Genchem Pharma Limited, Irvine, CA 92620-1998 (US)
(72) Inventor: MacDonald, Peter, 6925 Gentilino (CH); Rossetto, PierLuigi, 6828 Balerna (CH)
(74) Representative: Minoja, Fabrizio, Dr.

(57) **Abstract**

The invention provides a process for the preparation of (22S,22R)-9α-fluoro-11β-hydroxypregnane 16,17-acetal derivatives of formula (I) 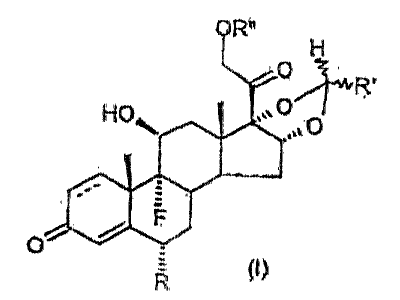 wherein: R is halogen, preferably fluorine, hydrogen or linear or branched C₁-C₄-alkyl; R' is linear, branched or cyclic C₁-C₁₂-alkyl, preferably n-propyl; R'' is hydrogen or C₁-C₁₆-acyl; a single or double bond bridges positions 1-2, which comprises reacting compounds (II) with compounds (III) in aqueous hydrofluoric acid, according to the following scheme 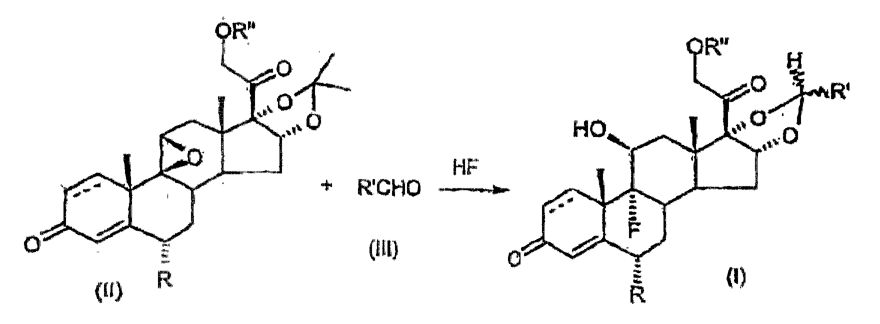 wherein R, R', R'' are as above defined.

## Description

The present invention relates to a process for the preparation of [22R,22S]-9α-fluoro- 11β-hydroxypregnane 16,17-acetal derivatives.

### Background of the invention

Certain (22R)-9α-fluoro-11β-hydroxypregnane 16,17-acetals have recently been found to possess very potent glucocorticoid receptor binding activity, as well as high rates of biotransformation (rapid inactivation), thus promising to provide steroids with an improved therapeutic index. Particularly promising examples of (22R)-9α-fluoro-11β-hydroxypregnane 16,17-acetals are Rofleponide [(22R)-6α,9-difluoro- 11β,16α,17,21-tetrahydroxypregn-4-ene-3,20-dione 16,17-butyraldehyde acetal] and Rofleponide 21-palmitate. (22R)-9α-fluoro-11β-hydroxypregnane 16,17-acetals are also useful intermediates for the manufacture of compounds such as Itrocinonide.

Rofleponide, as well as its (22S)-epimer, is disclosed in US 5674861, whilst processes for its synthesis are disclosed in US 5939409. Rofleponide palmitate is disclosed in US 5614514 and US 5888995. Itrocinonide is disclosed in US 4950659.

US 5939409 discloses three alternative methods for the preparation of Rofleponide (apart from simple hydrolysis of the corresponding 21-esters). These processes start from either: -
- the corresponding 16α,17α-diol,
- its 16,17-acetonide, or
- 1,2-dehydro-Rofleponide

All of these starting materials already contain the 9α-fluoro-11β-hydroxy-functionality but they are not readily available.

Moreover, all of the above processes for the synthesis of Rofleponide suffer from a number of shortcomings:
- synthesis from the corresponding 16α,17α-diol, as described in US 5939409, leads to a mixture of the both possible C-22 epimers and requires separation by the expensive technique of preparative chromatography.
- A second method, involving trans-acetalisation of the corresponding 16,17-acetonide, also leads to an epimeric mixture unless the reaction is conducted in a reaction medium that is a hydrocarbon. Although use of a hydrocarbon medium gives a somewhat better stereoselectivity for the preferred 22R-epimer, it requires that the reaction be conducted in the presence of a large amount of fine sand (20 kilograms/kilogram of starting material) and is therefore a process of extremely difficult industrial application.
- A third method, consisting of selective hydrogenation of 1,2-dehydro-Rofleponide, is characterised by a low yield, the need for preparative chromatography, as well as the need to prepare the starting material using the above-described "sand" procedure.

Starting materials employed in the above patents which contain only a double bond in the 4,5-position were obtained by selective hydrogenation of their corresponding 1,4-dienes, since some of these 1,4-dienes (e.g. fluocinolone acetonide) are commercially available. Such 1,4-dienes, however, are always produced commercially from more basic starting materials (such as diosgenin or sitosterol), which do not possess a double bond in the 1,2-position. This function has been introduced at considerable expense, and its subsequent removal is thus wasteful and expensive in terms of both reagents and loss of yield. A further disadvantage of such an approach is the need to use large quantities of a precious metal catalyst (such as tris(triphenylphosphine)rhodium) and the need to work with hydrogen gas (because of risks of explosion, this gas can only be used in special production areas).

### Description of the invention:

Now a "one-pot" process for the preparation of (22S,22R)-9α-fluoro-11β-hydroxypregnane 16,17-acetal derivatives, which overcomes the drawbacks encountered with known process, using cheaper and more readily available starting materials and allowing a simple and economic work-up and purification, has been found and it is the object of the present invention.

Specifically, the invention provides a process for the preparation of compounds (I): 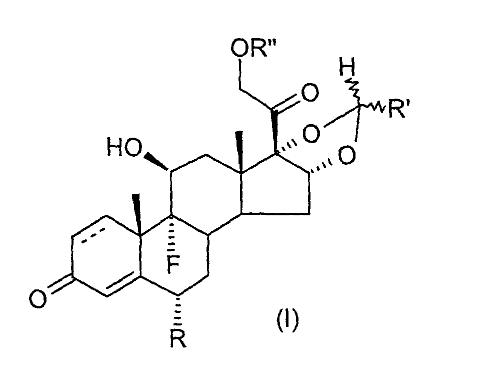 wherein: R is halogen, preferably fluorine, hydrogen or linear or branched C₁-C₄-alkyl; R' is linear, branched or cyclic C₁-C₁₂-alkyl, preferably n-propyl; R" is hydrogen or C₁-C₁₆-acyl; a single or double bond bridges positions 1-2, which comprises reacting compounds (II) with compounds (III) in aqueous hydrofluoric acid, according to the following scheme 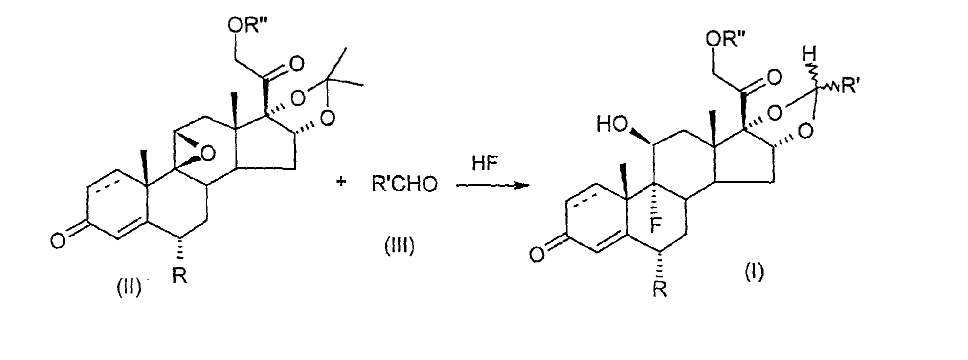 wherein R, R' and R" are as above defined.

The reaction is normally allowed to continue until fluorination and complete trans-acetalisation have taken place and the desired epimeric ratio is obtained. Initially, approximately equal amounts of 22-epimers are formed (kinetic control), whereas whenever the more thermodynamically stable (22R)-epimer is required, the reaction is simply allowed to continue until an equilibrium mixture of 22-epimers is obtained. The composition of such an equilibrium mixture, which typically contains 90% or more of the 22R-epimer, depends somewhat upon the reaction temperature, which can be varied from -70 to 10°C, preferably from -40 to -20 °C if the 22R-epimer is desired. From such equilibrium mixtures, pure 22R-epimers can be obtained in good yield by simple crystallisation, without any need for preparative chromatography. 22S-epimers present in the reaction mixture can in some cases be isolated in a pure state without any need to resort to expensive preparative chromatographic techniques.

In order to have a complete conversion of compounds (II) into (I), the molar ratio of compouns (III) to (II) is preferably ≥ 1. However, large excess of (III) is preferably avoided to prevent undesired side reactions.

Aqueous hydrofluoric acid is used as the solvent as well as reactant, at a concentration of 40-85%, preferably 65-75%. Optionally, an organic proton acceptor such as pyridine, tetrahydrofuran or aliphatic alcohol may also be present.

The starting compounds, 9β,11β-epoxy 16,17-acetonides, are readily available, e.g. according to the procedures described in US 4255331, which is hereby incorporated by reference.

Unlike the epimeric mixtures obtained by the process of US 5939409, the equilibrium mixtures obtained by the present process contain little or none of the corresponding acetonides (normally < 0.1%). Furthermore, in contrast to the process of US 5939409, the present process is conducted in solution, and no addition of sand to the reaction mixture is necessary. Because of the high dissolving power of the solvent/reagent used in the present process (concentrated hydrofluoric acid), the reaction can conveniently be carried out at quite high concentrations and, consequently, in smaller production equipment.

The process of the invention may be practiced on 9β,11β-epoxypregnane 16,17-acetonides having either an hydroxy or an acyloxy group in the 21-position. Preferably acetonides presenting a hydroxy group in position 21 are used as starting reagents. The product thus obtained according to the invention can then be acylated following conventional esterification techniques.

In a preferred embodiment of the invention, the 9β,11β-epoxy 16,17-acetonide used is 9β,11β-epoxy-6α-fluoro-16α,17,21-trihydroxypregn-4-ene-3,20-dione-16,17-acetonide, 9β,11β-epoxy-6α-fluoro-16α,17,21-trihydroxypregn-4-ene-3,20-dione-16,17-acetonide 21 acetate or 9β,11β-epoxy-6α-fluoro-16α,17,21-trihydroxypregn-4-ene-3,20-dione-16,17-acetonide 21 propionate, and the product is Rofleponide, Rofleponide 21-Acetate, or Rofleponide 21 propionate, respectively.

An advantage of the current process is that two (obligatory) steps in the overall manufacture of (22R,22S)-9α-fluoro-11β-hydroxypregnane 16,17-acetal derivatives, viz. hydrofluorination and acetal-formation, are carried out contemporaneously.

Although according to known processes (US 5939409) the starting materials already contain the 9α-fluoro-11β-hydroxy-functionality, these starting materials will inevitably have undergone, at some earlier stage in their manufacture, hydrofluorination of a 9β,11β-epoxy precursor. By carrying out both hydrofluorination and acetal-formation contemporaneously, the present invention leads to a significant increase in overall yield, as well as allowing savings due to reduced processing times, equipment utilisation, and reagent costs.

Furthermore, with known processes, the hydrofluorination of 9β,11β-epoxypregnanes is often accompanied by extensive side reactions (see Fried and Edwards, Organic Reactions in Steroid Chemistry, Chapter 8), and acceptable yields normally require that the reaction be carried out in the presence of an organic proton acceptor such as pyridine, tetrahydrofuran, or aliphatic alcohols. Surprisingly, in accordance with the process of the invention, fewer fluorination side products are formed and an essentially quantitative trans-acetalisation is obtained.

A further advantage of not isolating the intermediate 9α-fluoro-11β-hydroxypregnane 16,17-acetonides is that these intermediates are often less easily purified than are the corresponding 9α-fluoro-11β-hydroxypregnane 16,17-acetals.

According to a further embodiment, the present invention provides a method for the separation of the 22-epimers of the compounds of formula (I) by trituration/crystallisation. It has been found that both epimers can be obtained in pure form by carrying out selective dissolution of the 22R-epimer in methanol. Thus methanolic trituration of an epimeric mixture of compounds (I), followed by filtration, reduces the content of 22S-epimer in the filtrate to ca. 4-5%. Successive crystallisation of the filtrate, from lower C1-C3 alcohols or a mixture thereof with water, preferably from ethanol-water mixtures, enables pure 22R-epimers to be obtained in good yield. Furthermore, recrystallisation from anhydrous methanol of the undissolved solids, which consist mainly of 22S-epimer with a purity of up to 90%, enables pure (22S)-epimer to be easily obtained.

In a preferred embodiment, this process is used to separate 22-epimers of Rofleponide.

The invention is illustrated in more detail by the following examples.

### Example 1:

To 265 grams of hydrofluoric acid 72% at -40° was added 53 grams of 9β,11β-epoxy-6α-fluoro-16α,17,21-trihydroxypregn-4-ene-3,20-dione-16,17-acetonide (CAS RN 70141-43-4) followed by 9.15 grams (0.96 eq.) of butyraldehyde. After holding at -20° for 1 hour the reaction solution was poured into an ice cold mixture of water and sufficient ammonium hydroxide solution to give a final pH of 8.0. The resulting precipitate was collected and dissolved in dichloromethane and this solution was washed with water. The organic phase was evaporated to a small volume and diluted with toluene, whereupon Rofleponide (isomer ratio R/S 10:1) crystallised. Yield 46 grams. Two recrystallisations from aqueous ethanol afforded 99.0% pure Rofleponide.

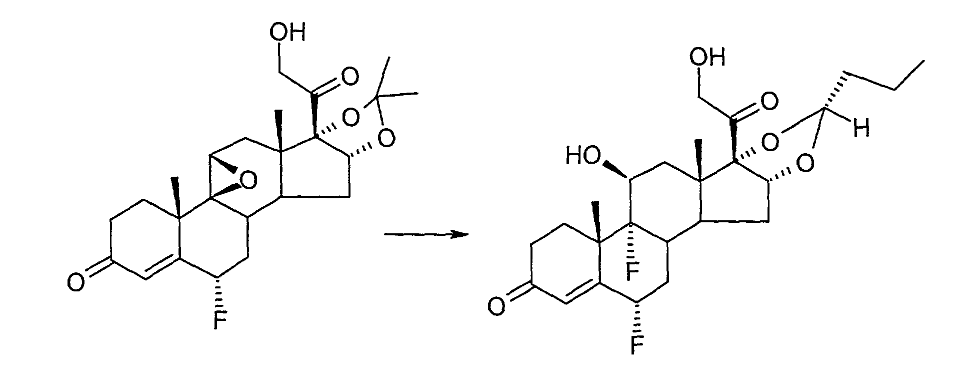

### Example 2:

5 To 200 grams of hydrofluoric acid 72% at -40° was added 50 grams of 9β,11β epoxy 6α fluoro 16α,17,21 trihydroxypregn 4 ene 3,20 dione 16,17 acetonide 21 propionate followed by 7.35 grams (1 eq.) of butyraldehyde. After holding at -20° for 2 hours the reaction solution was poured into an ice cold mixture of water and sufficient ammonium hydroxide solution to give a final pH of 8.0. The resulting precipitate was collected and dissolved in dichloromethane and this solution was washed with water. The organic phase was evaporated to a small volume and diluted with diisopropyl ether, whereupon Rofleponide 21 propionate (isomer ratio R/S 10:1) crystallised. Yield 42 grams. Two recrystallisations from aqueous ethanol afforded 99.0% pure R-epimer.

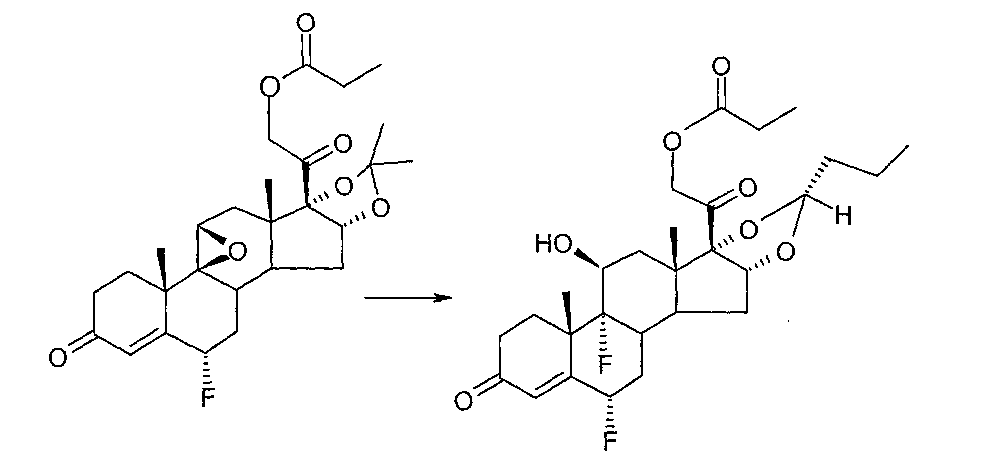

### Example 3:

To 200 grams of hydrofluoric acid 72% at -40° was added 50 grams of 9β,11β epoxy 16α,17,21 trihydroxypregn 4 ene 3,20 dione 16,17 acetonide, followed by 13.46 grams (1 eq.) of cyclohexanecarboxaldehyde. After being held at -20° for 2 hours the reaction solution was poured into an ice cold mixture of water and sufficient ammonium hydroxide solution to give a final pH of 8.0. The resulting precipitate was collected and dissolved in dichloromethane and this solution was washed with water. The organic phase was evaporated to a small volume and diluted with diisopropyl ether, whereupon 9α fluoro 11β,16α,17,21 tetrahydroxy pregn 4 ene 3,20 dione 16,17 cyclohexane-carboxaldehyde acetal (isomer ratio R/S 10:1) crystallised. Yield 50 grams. Two recrystallisations from aqueous ethanol afforded 99.0% pure R-epimer.

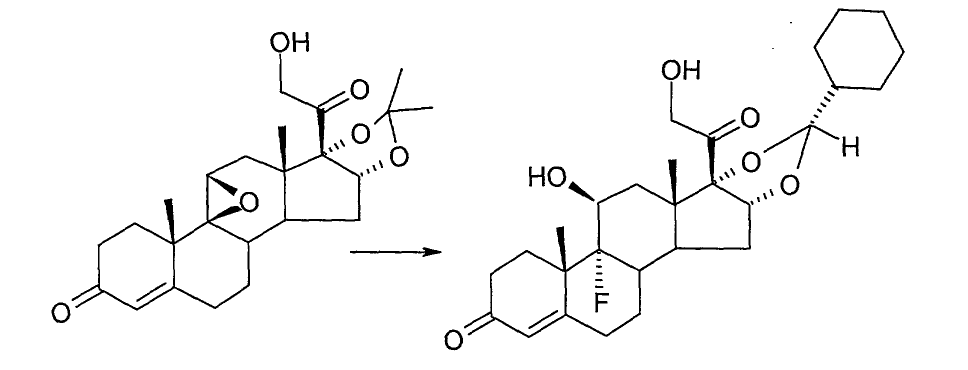

### Example 4:

To 150 grams of hydrofluoric acid 72% at -40° was added 30 grams of 9β,11β epoxy 6α fluoro 16α,17,21 trihydroxypregna 1,4 diene 3,20 dione 16,17 acetonide (purity 91%), followed by 5.16 grams (1 eq.) of butyraldehyde. After holding at the solution at 0° for 2 hours, then at -20° for 6 hours, the reaction solution was poured into an ice cold mixture of water and sufficient ammonium hydroxide solution to give a final pH of 8.0. The resulting precipitate was collected and dissolved in dichloromethane and this solution was washed with water. The organic phase was evaporated to a small volume and diluted with toluene, whereupon 1,2-Dehydro-rofleponide crystallised. Yield 28 grams, purity 98%, isomer ratio R/S 92:8). Two recrystallisations from aqueous ethanol afforded 99.0% pure R-epimer.

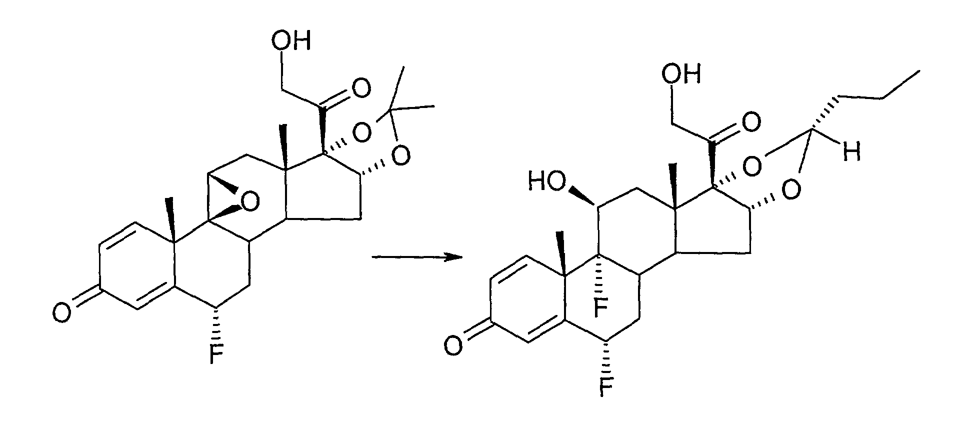

### Example 5 - separation of isomers of Rofleponide:

Rofleponide (42.4 grams of equilibrium mixture, isomer ratio R/S 10:1) was added to methanol (175 grams) containing 0.85 grams of acetic acid. The suspension was stirred for 60 minutes at room temperature and filtered through a pad of Hyflo Super Cel, rinsing with a further 48 grams of methanol.

HPLC analysis showed an isomer ratio R/S 19:1 in the filtrate, whilst the solids contained mainly 22S epimer (isomer ratio R/S 1:10).

After dilution of the filtrate with water, and recrystallisation (ethanol-water) of the resulting precipitate, 99% pure Rofleponide was obtained.

Recrystallisation (anhydrous methanol) of the solids from the trituration lead to 99% pure 22S-epimer.

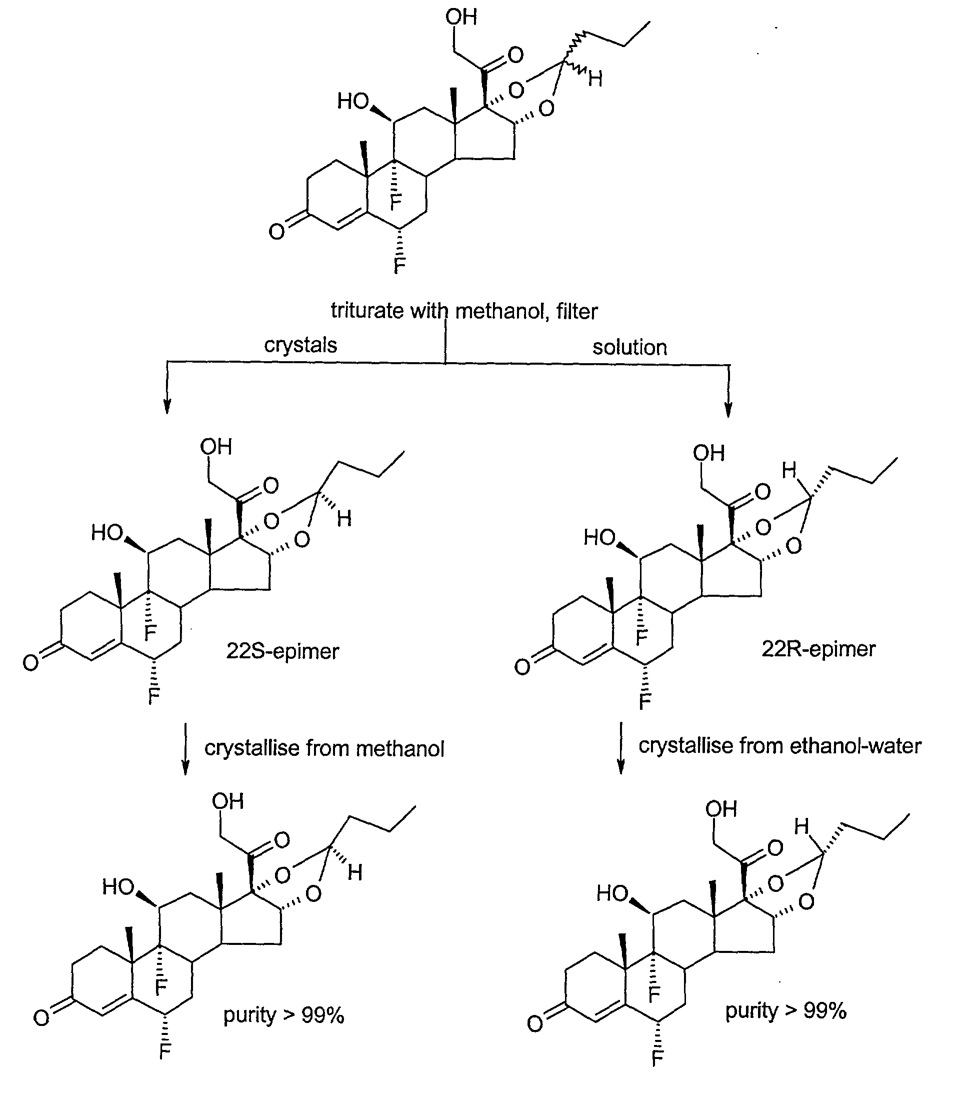

## Claims

1. A process for the preparation of compounds of formula (I): 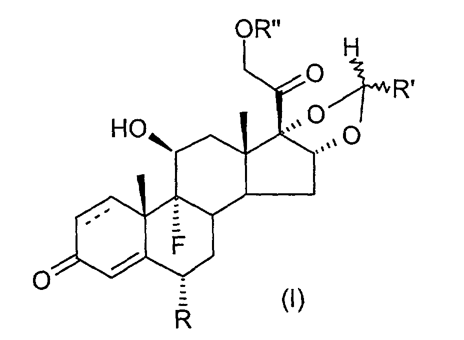 wherein: R is selected from halogen, hydrogen, linear or branched C₁-C₄-alkyl; R' is selected from linear, branched or cyclic C₁-C₁₂-alkyl; R" is selected from hydrogen or C₁-C₁₆-acyl; a single or double bond bridges positions 1-2, which comprises reacting compounds (II) with compounds (III) in aqueous hydrofluoric acid, according to the following scheme:

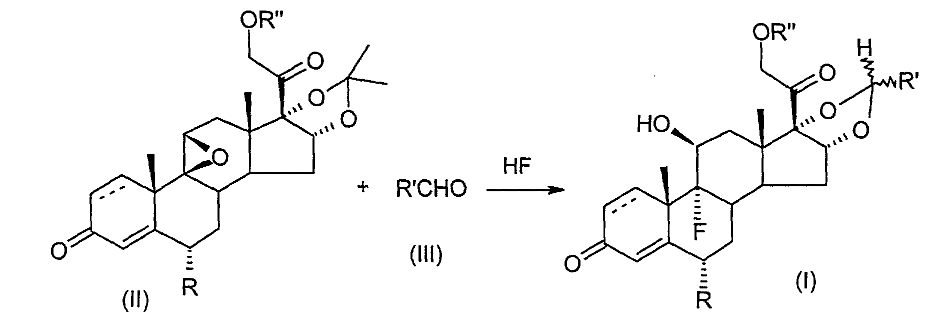

2. A process according to claim 1, wherein R is fluorine, R' is n-propyl and a single bond bridges positions 1-2.

3. A process according to claims 1-2, wherein the reaction temperature is from -70 to 10 °C.

4. A process according to claim 3, wherein the temperature is from -40 to -20°C.

5. A process according to claims 1-4, wherein the molar ratio of compounds (III) to compounds (II) is ≥ 1.

6. A process for the separation of the 22-epimers of compounds (I), which comprises the following steps:
a) methanolic trituration of an epimeric mixture of compounds (I),
b) filtration of the methanolic mixture from (a);
c) crystallisation of the filtrate using C1-C3 alcohols or a mixture thereof with water as crystallisation solvent, to isolate the 22-R-epimer;
d) optionally, crystallisation from anhydrous methanol of the undissolved solid from (b) to isolate the 22-S-epimer.

7. A process according to any of the previous claims, for the preparation of a compound selected from the group consisting of:
a) (22R)-6α,9-difluoro-11β,16α,17,21-tetrahydroxypregn-4-ene-3,20-dione 16,17-butyraldehyde acetal;
b) (22R)-6α,9-difluoro-11β,16α,17,21-tetrahydroxypregn-4-ene-3,20-dione-21-propionate-16,17-butyraldehyde acetal;
c) (22R)-6α,9-difluoro-11β,16α,17,21-tetrahydroxypregn-4-ene-3,20-dione 16,17 cyclohexane carboxyaldehyde acetal;
d) (22R)-6α,9-difluoro-11β,16α,17,21-tetrahydroxypregna-1,4-diene-3,20-dione 16,17-butyraldehyde acetal.
